# EUROPEAN PATENT APPLICATION

(11) **EP 4 659 663 A1**
(43) Date of publication of application: **10.12.2025**
(21) Application number: 24180611.6
(22) Date of filing: 06.06.2024
(51) Int. Cl.: A61B 5/021, A61B 5/026, A61B 5/0295

(54) **A DEVICE AND METHOD FOR MONITORING BLOOD PRESSURE OF A SUBJECT BY PERFORMING MEASUREMENTS ON A TISSUE OF THE SUBJECT**

(71) Applicant: Nederlandse Organisatie voor toegepast-natuurwetenschappelijk Onderzoek TNO, 2595 DA 's-Gravenhage (NL)
(72) Inventor: AKKERMAN, Hylke Broer, 2595 DA 's-Gravenhage (NL); KHATOUN, Ahmad, 2595 DA 's-Gravenhage (NL)
(74) Representative: V.O.

(57) **Abstract**

The invention pertains to a device designed for non-invasive monitoring of blood pressure by measuring changes in blood volume within a subject's tissue. The device includes multiple light sources and photodiodes, organized into groups. Each group consists of at least one light source and multiple photodiodes placed at various distances from the light source. This configuration allows for the detection of reflected light that carries photoplethysmography (PPG) signals from different depths within the tissue. The device features a processing unit that processes the PPG signals to perform signal separation analysis, distinguishing the signals from various tissue depths. The processing unit also calculates pulse wave velocity (PWV) by examining the time difference between detections at photodiodes located at different distances. This PWV is subsequently used to estimate the subject's blood point pressure using a predetermined correlation between PWV and blood pressure.

## Description

### FIELD OF THE INVENTION

The invention relates to a device for monitoring blood pressure of a subject by performing measurements on a tissue of the subject. Furthermore, the invention relates to a method for monitoring blood pressure in a subject by analyzing tissue of the subject. The invention also relates to a use of the device for monitoring blood pressure of a subject. Also, the invention relates to a system comprising a device for monitoring blood pressure of a subject.

### BACKGROUND TO THE INVENTION

Blood pressure is an important vital sign, providing essential insight into cardiovascular health and the state of other bodily functions. Accurate and continuous monitoring of blood pressure is vital, especially for individuals suffering from cardiovascular diseases or those who are critically ill.

Traditionally, blood pressure is measured non-invasively using a sphygmomanometer, which employs an inflatable cuff to restrict blood flow. While this method is recognized for its reliability, it presents numerous drawbacks, particularly when continuous monitoring is required.

The limitations of the sphygmomanometer include its intrusiveness, discomfort to the patient, and the impracticality for long-term and ambulatory monitoring. These drawbacks have fueled the pursuit of alternative technologies for non-invasive, continuous blood pressure monitoring. To date, various devices and methodologies have been explored, yet each presents its own set of challenges and limitations.

The most common contemporary methods integrate electrocardiography (ECG) and photoplethysmography (PPG) to estimate blood pressure. This approach, often employed in wearable technologies such as wristbands and smartwatches, calculates the pulse arrival time (PAT) from the heart to the measurement site. PAT is derived from the pre-ejection period (PEP) and the pulse transit time (PTT), the latter being a function of the local pulse wave velocities in the body. Although widely used, this method introduces significant inaccuracies due to the averaging of varied pulse wave velocities and the influence of factors such as arterial elasticity and diameter, as well as the presence of arterial bifurcations.

Another emerging method utilizes machine learning techniques applied to PPG waveforms. These methods attempt to enhance the accuracy of blood pressure estimations by analyzing complex signal patterns. However, the success of such approaches is heavily dependent on the quality of the data and the sophistication of the algorithm, which may vary widely in real-world conditions.

Despite these innovations, the need for a more reliable and accurate method for continuous and non-invasive blood pressure monitoring remains unmet. The complexity of the vascular system and the dynamic nature of blood flow continue to pose significant challenges in accurately detecting and measuring pulse waves and translating these into precise blood pressure readings.

The existing technologies, while advancing the field, still struggle with issues of signal degradation due to motion artifacts, noise, and the inherent limitations of sensor technologies that fail to capture the detailed dynamics of deep arterial blood flow. These challenges underscore the ongoing desire for an improved device for blood monitoring that can deliver the precision and reliability necessary for effective monitoring and management of cardiovascular health.

### SUMMARY OF THE INVENTION

It is an object of the invention to provide for a method and a system that obviates at least one of the above mentioned drawbacks.

Additionally or alternatively, it is an object of the invention to provide for an improved method and device for monitoring blood pressure of a subject.

Additionally or alternatively, it is an object of the invention to enhance the accuracy and reliability of blood pressure measurements by focusing on deeper arterial signals, isolated from superficial vascular activities.

Additionally or alternatively, it is an object of the invention to facilitate continuous, non-invasive blood pressure monitoring using a device that minimizes discomfort and user intervention.

Additionally or alternatively, it is an object of the invention to deliver an improved analytical system that utilizes signal processing techniques to improve the detection of vital cardiovascular signals under varying physiological conditions.

Thereto, the invention provides for a device for monitoring blood pressure of a subject by performing measurements on a tissue of the subject, comprising: a plurality of light sources and photodiodes, wherein the light sources are configured to emit light and the photodiodes are configured to detect light reflected from the tissue of the subject, wherein reflected light includes photoplethysmography, PPG, signals indicative of blood volume changes, wherein the photodiodes and light sources are arranged into at least two groups, each group comprising at least one light source and at least two photodiodes positioned at varying distances from a corresponding light source within the respective group; a processing unit configured to process the PPG signals received by the photodiodes, and wherein the processing unit is configured to perform signal separation analysis in order to separate PPG signals originating from different depths within the tissue of the subject; wherein the processing unit is configured to calculate a pulse wave velocity (PWV) based on the separated PPG waveforms associated to deeper arteries by analyzing the time difference between signal detections at the photodiodes positioned at varying distances; and wherein the processing unit is configured to determine a blood pressure of a subject based on the calculated PWV utilizing a predetermined relationship between PWV and blood pressure.

The device configured for monitoring blood pressure utilizes a configuration that includes multiple light sources and photodiodes, wherein these components are specifically arranged in at least two groups, each comprising at least one light source and two photodiodes at varied distances. Such arrangement allows for the detection of PPG signals that are reflective of blood volume changes at different tissue depths. The processing unit of the device is adept at handling signal separation analysis to distinctly identify PPG signals from superficial and deeper tissue layers, thereby enabling the precise calculation of pulse wave velocity (PWV) based on the timing differences of signals detected at different distances. This arrangement significantly enhances the accuracy of non-invasive blood pressure measurements by directly correlating the determined PWV with established blood pressure values, overcoming limitations of traditional single-point measurement systems.

The arrangement of the light sources and photodiodes into at least two distinct groups, with each group containing at least one light source and a minimum of two photodiodes positioned at different distances from the corresponding light source, provides for a spatial distribution that is specifically configured to capture photoplethysmography (PPG) signals that vary due to changes in blood volume occurring at multiple depths within the tissue. The multiple-depth capability can be important because it enables the device to capture a broader spectrum of cardiovascular activity, providing a more comprehensive picture of blood flow dynamics. Each group of light sources and photodiodes works to emit and detect light that is reflected back from varying depths, with the distance of each photodiode from its light source configured to optimize the detection of light reflected from specific tissue layers.

The processing unit is capable of performing signal separation analysis. This analysis can be important for accurately distinguishing the PPG signals obtained from different tissue depths. The processing unit can isolate specific PPG signals that correspond to distinct blood flow characteristics associated with different vascular structures. This capability to differentiate signals can be important for reducing the noise and enhancing the clarity of the data obtained, which is often a challenge with traditional PPG-based devices.

Advantageously, a precise calculation of pulse wave velocity (PWV) can be obtained. PWV is an important indicator of arterial stiffness and is directly related to blood pressure. By analyzing the time differences between PPG signals detected by photodiodes at varying distances, the device calculates the velocity of the blood pulse as it travels through the arteries. This calculation is more refined compared to traditional methods due to the improved signal resolution and the ability to specifically attribute time shifts to changes in pulse velocity rather than variations in signal quality or external interference.

The ability to accurately correlate the calculated PWV with established blood pressure values is a significant enhancement over existing single-point measurement systems. This correlation is facilitated by the precise and differentiated capture of PPG signals, allowing for a direct and reliable estimation of blood pressure based on physiological relationships between PWV and blood pressure levels. This integration of sensor arrangement with signal processing and analysis provides a robust framework for non-invasive, continuous, and accurate blood pressure monitoring, addressing many of the limitations found in previous technologies.

Optionally, the signal separation analysis is a multivariate signal processing.

The device can employ multivariate signal processing as a form of signal separation analysis, which is integral for effectively differentiating between PPG signals derived from multiple depths within the subject's tissue. This signal processing capability enables the device to handle complex data sets where signals from different tissue depths may overlap, ensuring a more accurate and reliable analysis. The effect of using multivariate signal processing is an enhanced clarity and separation of signals, which directly contributes to the overall precision and reliability of the PWV and blood pressure measurements.

This form of processing is particularly valuable in the context of photoplethysmography (PPG) where signals derived from multiple depths within the tissue can significantly overlap due to the simultaneous presence of blood volume changes occurring at various tissue layers. The overlapping of signals can lead to ambiguous data, which, if not properly resolved, can result in inaccurate blood pressure readings. Multivariate signal processing, however, is equipped to analyze and manage these complex datasets effectively.

The key advantage of employing multivariate signal processing lies in its ability to disentangle these overlapped signals by treating the signal data as a composite of multiple variables. Each variable represents a component of the overall signal that can be attributed to a specific depth within the tissue. By applying algorithms that analyze variations across multiple dimensions or variables simultaneously, this processing technique can differentiate signal components with greater precision than univariate analyses, which may only consider single data points without regard to their interactions with other concurrent data points.

The effect of using multivariate signal processing is an enhanced clarity and separation of signals. Through this improved signal resolution, the processing unit can distinctly identify and separate the PPG signals associated with superficial blood vessels from those originating from deeper arterial structures. Such clarity can be important for accurate pulse wave velocity (PWV) measurement because PWV calculations require precise identification of the timing of blood flow at specific depths to accurately assess the speed at which the pulse wave travels through the arteries.

Moreover, the separation of these signals mitigates the risk of signal contamination that can occur when signals from different depths are inaccurately merged or confused. This clear segregation of data ensures that the PWV calculated reflects a more accurate physiological measurement of arterial stiffness and blood pressure, rather than an erroneous interpretation caused by overlapping data.

The reliable differentiation and analysis of PPG signals through multivariate signal processing not only enhance the precision of PWV calculations but also improve the overall reliability of the non-invasive blood pressure measurements provided by the device.

Optionally, the signal separation analysis is an independent component analysis.

Independent component analysis (ICA) is particularly advantageous for isolating and identifying individual PPG signals from mixed signal data, which can be important for accurately measuring the PPG signals originating from deeper arterial vessels. The effect of employing ICA is the increased accuracy in the isolation of signal components specific to different arterial depths, thereby improving the precision of PWV calculations and subsequently, blood pressure determinations.

ICA is a statistical and computational technique configured to decompose a multivariate signal into additive, independent non-Gaussian signals. This capability is especially beneficial where signals from biological tissues overlap and are contaminated by various noise sources.

The ICA can be used to enable effective distinction between PPG signals that originate from various depths within the tissue. Since the PPG signal is reflective of blood volume changes, which vary depending on the depth of the blood vessels from which they are sourced, the ability to isolate these signals can be important. Deeper arteries, which are of particular interest for accurate pulse wave velocity (PWV) measurement, present a significant challenge due to the attenuation and mixing of signals as they pass through multiple tissue layers.

The ICA can significantly improve the clarity of PPG signals by effectively separating them based on their statistical independence. This separation can be important in applications where signals from superficial and deeper tissue layers need to be distinguished without physical segregation of the source or sensors.

By isolating components that specifically represent the blood flow dynamics of deeper arterial vessels, ICA enables the device to more accurately assess the PPG signals pertinent to those vessels. This is particularly important for PWV measurement, as PWV is a key parameter in determining blood pressure and is predominantly influenced by the condition and dynamics of major arteries.

Often, PPG signals are subject to interference from various sources, such as motion artifacts or ambient light changes. ICA helps in identifying and excluding these artifacts from the signals of interest, thus improving the reliability of the readings.

With clearer and more specifically targeted PPG signals, the time shift measurements required to calculate PWV can be made more precisely. Since PWV is calculated by assessing the time it takes for the blood pulse to travel between points, the accuracy of identifying these specific points through clean, isolated signals can be important.

The measurement of PWV allows to determine the blood pressure of the subject accurately. By improving the precision of PWV calculations through better signal analysis via ICA, the overall accuracy and reliability of blood pressure measurements are significantly enhanced.

Optionally, the processing unit is configured to calculate the PWV based on time shifts between the separated PPG signals corresponding to arterial blood flow from deeper lying arteries as detected by photodiodes across different groups, and wherein the processing unit is configured to perform the calculation taking into account known distances between the photodiodes and their respective light sources in the different groups and/or specific arrangement of photodiodes in order to capture the travel path of the pulse wave.

By incorporating known distances between photodiodes and their corresponding light sources, as well as specific arrangements of these components, the device can precisely track the travel path of the pulse wave across the tissue. This approach allows for an enhanced accuracy in PWV measurement, directly translating to more reliable blood pressure monitoring. The arrangement and known distances ensure that the pulse pathway is accurately captured, thus improving the fidelity of the PWV determination.

The device calculates PWV by considering the time shifts observed in PPG signals that are separated based on their depth within the tissue, particularly focusing on signals from deeper lying arteries. These time shifts are important as they represent the delay between pulse wave detections at different locations, directly affected by the speed of the blood flow which in turn is influenced by the arterial blood pressure.

The use of known distances between the photodiodes and their respective light sources within the device, enables the device to accurately model and trace the actual path taken by the arterial pulse wave as it travels through the tissue. The precise arrangement of these components plays a significant role in ensuring that the time shift measurements are not only accurate but also relevant to the actual physiological pathways of arterial blood flow. For instance, arranging the photodiodes at strategic distances that align with the expected trajectory of the pulse wave through the major arteries enhances the fidelity of the captured data.

By leveraging the known spatial arrangements and the detected time shifts, the device can calculate the PWV with enhanced precision. This precision stems from the ability to directly correlate specific time delays with the exact distances they cover, which can be important for accurate PWV calculation. The accurate calculation of PWV is directly linked to reliable blood pressure measurements.

Optionally, the device employs a plurality of light wavelengths in order to differentiate and separate PPG signals from superficial and deeper arteries based on the absorption characteristics of the tissue.

Different wavelengths have distinct absorption characteristics in tissue, which facilitates the differentiated detection of blood volume changes at various depths. This capability allows the device to effectively target specific tissue layers, enhancing the specificity and accuracy of the data collected, which can be important for accurate PWV measurement and, consequently, precise blood pressure monitoring.

The use of different wavelengths of light, each with unique penetration abilities and tissue absorption characteristics, allows the device to capture PPG signals from varied tissue depths more distinctly. Visible light tends to be absorbed more by superficial layers, while near-infrared light penetrates deeper, reaching the larger arteries. This differentiation can be important because it enables the device to selectively target and analyze the blood volume changes specific to different vascular layers.

By capturing PPG signals from both superficial and deeper tissues, the device can more accurately compute the pulse wave velocity (PWV). Since PWV is a key indicator of arterial stiffness and thereby blood pressure, the ability to assess the PWV from deeper arteries, where the pulse wave dynamics are more indicative of true vascular health, enhances the reliability of the blood pressure readings.

The application of multiple wavelengths enables the device to effectively filter and separate the PPG signals according to depth, which significantly reduces the cross-signal interference often seen in conventional single-wavelength PPG devices. This separation is achieved because the different wavelengths are absorbed differently by various biological components (like oxyhemoglobin and deoxyhemoglobin), which vary in concentration between superficial and deeper tissue layers. Consequently, the device can isolate signals with higher specificity, leading to more sensitive and accurate assessments.

Shorter wavelengths, which are more readily absorbed by the upper layers of the skin, provide information about the blood flow in superficial capillaries. Longer wavelengths, particularly in the near-infrared spectrum, penetrate deeper, bypassing the superficial layers to reach the major arteries. Here, they provide valuable data on the blood flow dynamics that are more representative of central arterial behavior. These differentiated signals are then detected by the photodiodes, and the processing unit employs processing steps to analyze these signals separately, thereby calculating the PWV for each targeted tissue layer.

Optionally, each light source is configured to emit light in both the visible and near-infrared spectrum.

This enables versatile and effective probing of different tissue depths. Visible light is generally absorbed superficially, whereas near-infrared light penetrates deeper, allowing for a comprehensive analysis of blood volume changes across various layers of the tissue. This multiple-wavelength approach maximizes the effectiveness of the device in capturing a complete spectrum of PPG signals, thus ensuring a robust analysis for PWV and blood pressure determination.

By utilizing both visible and near-infrared light, the device can capture a complete spectrum of PPG signals from both superficial and deeper tissue layers. This comprehensive capture can be important for constructing a detailed and accurate profile of blood flow dynamics across different vascular layers, which can be important for the subsequent analysis of pulse wave velocity (PWV) and blood pressure.

The ability to differentiate between PPG signals originating from various depths enhances the precision with which PWV is calculated. Since PWV is an important parameter in determining blood pressure, the accuracy of its measurement directly influences the reliability of the blood pressure readings obtained by the device. By having access to distinct PPG signals from both superficial and deep tissues, the device can more accurately assess the time it takes for the pulse wave to travel between sensors, leading to a more accurate calculation of PWV.

The multiple-wavelength (e.g. dual-wavelength) approach minimizes the impact of potential interferences that can affect the PPG signal quality, such as ambient light, tissue composition, and changes in external conditions. By analyzing the signals obtained from both visible and near-infrared light, the device can perform a robust analysis, distinguishing genuine blood flow changes from noise and artifacts. This robustness can be important for ensuring the reliability of the health metrics being monitored.

The variability in tissue composition among different patients can significantly affect the penetration and absorption of light. With the capability to emit both visible and near-infrared light, the device can adapt to individual differences in tissue characteristics, ensuring effective monitoring across a broad population. This adaptability is particularly beneficial in clinical settings where patient variability is a common challenge.

Optionally, a first distance (D3) is defined as the distance between a light source in the first group of photodiodes and the nearest photodiode in a second group, wherein a second distance (D2) is defined as the distance between the furthest photodiode and the light source within that specific group, wherein the first distance is greater than any instance of D2 within either of the groups.

A specific spatial configuration may be employed where the distance between a light source in the first group of photodiodes and the nearest photodiode in a second group (D3) is significantly greater than the distance between the furthest photodiode and the light source within any group (D2). This spatial arrangement can be important for ensuring that the time shift measurements for PWV calculation are not confounded by closely spaced measurements, which can reduce the accuracy of the results. The increased D3 distance facilitates a clearer distinction in timing between PPG signals detected by photodiodes in different groups, leading to more precise and reliable PWV calculations.

By setting a greater distance (D3) than any within-group distance (D2), the device minimizes the potential for signal overlap or interference. In pulse wave velocity measurements, the clarity of the time shift between signals detected at different photodiodes can be important. If photodiodes are too closely spaced, the pulse wave detected may not have sufficient space to demonstrate measurable changes in velocity, which can confuse the data and lead to inaccurate PWV calculations. By enlarging D3, the device ensures that the pulse wave has adequate travel distance to manifest detectable changes in velocity by the time it reaches the next group of photodiodes.

The increased distance (D3) enhances the timing resolution between PPG signals detected by photodiodes across different groups. This configuration allows the device to more accurately capture the timing differences as the pulse wave travels from one group to the next. Such precision in measuring the time shifts directly impacts the reliability of calculated PWV values. Since PWV is inversely related to arterial elasticity, which in turn is correlated with blood pressure, precise PWV measurements are important. This configuration directly translates into more reliable blood pressure readings by ensuring that the pulse wave's transit time, and thus its velocity, is measured over a sufficient spatial extent to reflect true physiological conditions.

Furthermore, this configuration helps in mitigating issues related to environmental noise and variations in tissue composition, which may otherwise distort the signal if the photodiodes were placed closer together. By spacing the groups of photodiodes, the device effectively isolates the pulse signals pertinent to deeper arterial blood flow from those associated with superficial blood flow or other noise factors.

Hence, by maintaining a greater inter-group distance compared to within-group distances, accuracy and reliability of the device assessments can be improved.

Optionally, the first distance is at least twice the second distance.

Advantageously, the device's capability to accurately measure pulse wave velocity (PWV) can be ensured by detecting minimal time shifts in photoplethysmography (PPG) signal arrival across the photodiodes.

The first distance (D3) being at least twice the second distance (D2) ensures a significant separation between the groups of photodiodes, which enhances the device's sensitivity to changes in the pulse wave velocity. This configuration minimizes the potential for signal interference and improves the temporal resolution of PPG signal detection, both of which are important for achieving high accuracy in the calculation of PWV and thus blood pressure.

By defining that the first distance (D3), which is the distance from a light source in the first group of photodiodes to the nearest photodiode in a second group, is at least twice the distance of the second distance (D2), which is the distance between the furthest photodiode and the light source within any group, the claim sets a minimum spatial criterion that has significant implications for the performance of the device.

The primary advantage provided by this specific spatial arrangement is the enhancement of the device's sensitivity to changes in pulse wave velocity (PWV). By ensuring a substantial separation (D3 being at least twice D2), the device is better equipped to capture the true propagation of pulse waves between the groups, free from the distortions that may result from more closely spaced diode arrangements.

One of the important challenges in accurately measuring PWV using photoplethysmography signals is the potential for signal interference. This interference can arise from overlapping signal pathways where photodiodes are too closely placed. With D3 significantly greater than D2, the signals captured by the photodiodes in different groups are less in some examples to suffer from spatial and temporal overlap, thereby reducing the noise and improving the clarity of the signal data used for PWV calculations.

The setting/configuration of D3 being at least twice D2 ensures that the time difference between the detection of pulse waves at various photodiodes can be captured more distinctly. When photodiodes are too close to each other relative to the speed of the pulse wave, the temporal differences between signal detections can be too subtle, making it difficult to accurately calculate the PWV. The specified distance ratio enhances the temporal resolution of the signal detection, allowing the device to more precisely determine the time it takes for a pulse wave to travel from one group of photodiodes to another.

The spatial configuration described minimizes potential errors in PWV calculation that arise from inadequate separation of measurement points. By ensuring that the pulse wave's travel between measurement points is sufficiently long (due to D3 being significantly larger than D2), the system can more accurately measure the velocity of the pulse waves. This accuracy can be important because PWV is directly used to calculate blood pressure; hence, more precise PWV measurements lead to more accurate blood pressure readings.

Optionally, the device is configured to determine a value indicative of an arterial direction at or approximate a location where the device is configured to perform blood pressure monitoring.

This can be utilized for aligning the device's sensors with the main arterial flow. This alignment can be important for accurate PWV measurement, as it ensures that the sensors are positioned in a manner that optimally captures the directional blood flow, thereby minimizing errors related to angular misalignment of the sensors relative to the arterial path. The ability to determine arterial direction enhances the overall accuracy and reliability of the blood pressure monitoring process.

Misalignment of sensors relative to the arteries can lead to errors in measuring the time it takes for the pulse wave to travel between two points (PWV), thus distorting the blood pressure reading. By incorporating a feature that determines the arterial direction, this device mitigates these risks, aligning the sensors so that they are positioned parallel to the arterial flow.

The device may be configured to include sensors that can detect not just the presence of blood flow but also the directionality of this flow. This is achieved through the strategic placement and orientation of photodiodes and light sources, which can sense the directional pattern of blood flow within the arteries. By understanding where the arteries are located and how they are oriented, the device can adjust the placement or orientation of its sensors accordingly.

Accurate alignment with arterial flow ensures that the PWV is measured along the true path of the arterial pulse wave, rather than across an incorrect axis which can falsely represent the velocity of the pulse wave. This alignment can be important for reducing angular errors or discrepancies that occur when the pulse wave's travel path and the sensor's measurement axis do not align.

By ensuring that the measurement sensors are oriented correctly with respect to the arterial flow, the device captures a more accurate representation of the pulse wave. This fidelity can be important for using PWV to calculate blood pressure, as PWV is highly dependent on the biomechanical properties of the arteries, which vary depending on the orientation of the arteries and the pressure exerted by the arterial walls.

Optionally, the device includes an array of photodiodes arranged to allow for the determination of the arterial direction by analyzing spatial variations in an AC/DC ratio of the PPG signals.

An array of photodiodes is configured to analyze spatial variations in the AC/DC ratio of PPG signals, which assists in determining the arterial direction. This capability allows the device to adjust sensor placement for optimal alignment with the arterial flow, significantly enhancing the precision of PWV calculations. This technical configuration ensures that the measurements are conducted in a manner that is most conducive to capturing the true dynamics of arterial blood flow, thereby improving the accuracy of blood pressure estimation.

PPG signals, which reflect blood volume changes, contain both alternating current (AC) and direct current (DC) components. The AC component is indicative of pulsatile blood flow changes due to cardiac cycles, whereas the DC component is influenced by the baseline blood volume and tissue characteristics. By analyzing the ratio of these two components (AC/DC ratio), the device can infer changes in blood flow characteristics, including flow directionality.

This analysis can be important because the arterial blood flow has a main direction, which ideally should align with the sensor array for optimal signal capture. Misalignment can lead to distorted PPG readings due to the angle of blood flow relative to the sensor orientation, which may affect the accuracy of derived metrics like PWV.

The precise determination of arterial direction allows the device to adjust the positioning of the photodiodes so they are optimally aligned with the arterial flow. This alignment can be important for capturing the true velocity of the pulse wave as it travels along the artery. Accurate detection of the time difference in pulse wave arrival between sensors directly influences the reliability of PWV calculations. When sensors are aligned with the blood flow, the measurements of pulse transit time are more accurate, leading to a more reliable estimation of blood pressure.

The capability of the device to analyze spatial variations in the AC/DC ratio not only aids in the initial determination of arterial direction but also allows for ongoing adjustments in sensor placement in real-time applications.

The configuration of the photodiode array to analyze the AC/DC ratio enables a dynamic response to the physiological and anatomical variability of subjects, which in turn supports a more tailored and individualized approach to non-invasive blood pressure monitoring. By adapting to the specific arterial characteristics of each subject, the device can optimize its measurements for enhanced accuracy and reliability, thus providing a significant improvement over traditional, less adaptive blood pressure monitoring systems.

Optionally, the determination of arterial direction is utilized to refine the calculation of PWV by ensuring a measurement path substantially aligns with a main directionality of arterial flow.

Geometric uncertainties in PWV calculation can be effectively reduced.

Utilizing the determination of arterial direction to refine PWV calculations ensures that the measurement pathway is substantially aligned with the main arterial directionality. This alignment can be important for accurate PWV measurements as it reduces discrepancies that may arise from measuring across misaligned paths, thereby significantly enhancing the precision and reliability of the blood pressure values obtained. This effect leverages geometric analysis to ensure that the pulse wave travel path is correctly captured, which directly improves the fidelity of the calculated blood pressure.

The alignment of the measurement pathway with the arterial direction minimizes errors that can arise from cross-sectional or tangential measurements relative to the artery's path. Such misalignments can lead to inaccuracies because the pulse wave's travel time may be captured over a path that does not accurately represent the arterial length, resulting in erroneous PWV and thus, blood pressure estimations.

By determining the arterial direction at the site of measurement, the device can adjust the placement of photodiodes and light sources to align with this direction. This strategic alignment ensures that the sensors capture the pulse wave longitudinally along the artery, rather than across it, which can be important for precise timing measurements.

Accurately aligning the device with the artery's main flow direction allows for a more direct measurement of the pulse wave's travel time between two points along its natural path. This can be important because any deviation from this path can introduce latency in the signal detection that is not due to the pulse wave's natural velocity, but rather due to the increased path length caused by improper sensor alignment. By reducing these geometric discrepancies, the device can offer more reliable and consistent PWV readings.

Optionally, the device is configured to analyze PPG signals and spatial data from the photodiodes and light sources, to calculate the arterial direction at or approximate to the location on a body of the subject where blood pressure monitoring is performed, wherein the processing unit is configured to distinguishing PPG signal characteristics that vary with arterial blood flow direction, and employ a geometric analysis based on the relative positions of photodiodes and light sources to deduce the orientation of the underlying arteries, and wherein the device includes or is connectable to an interface for displaying or communicating the determined arterial direction in order to assist in placement and orientation of the device for blood pressure monitoring.

The device can include capabilities for combining PPG signal and spatial data analysis to accurately determine arterial direction, employing geometric analysis of the relative positions of photodiodes and light sources. This determination can be important for optimal device placement and orientation, which enhances the accuracy of blood pressure monitoring by ensuring that the measurement sensors are properly aligned with arterial flow. The inclusion of an interface for displaying or communicating the determined arterial direction aids in the practical application of the device, making it user-friendly and effective in clinical settings.

By employing geometric analysis on these relative positions, the device can accurately ascertain the direction of arterial blood flow within the area being monitored. The ability to determine arterial direction ensures that the device's sensors are aligned parallel to the arterial flow, minimizing angular discrepancies which can significantly distort the measurement of pulse transit times and pulse wave velocities.

Accurate determination of arterial direction allows for strategic placement of the device on the body, which optimizes the reception of PPG signals that are most reflective of true arterial behavior. This strategic placement directly translates to more precise and less noisy signal data.

By analyzing the spatial positioning of photodiodes and light sources, the device can integrate the directional data with the PPG signals obtained. This integration can be important for differentiating PPG signals based on their origin, whether from superficial or deeper arterial layers, and their alignment with the arterial flow.

The geometric analysis allows the device to correct or adjust the measurement path, ensuring that it closely mirrors the actual path of the arterial blood flow, thus improving the quality and reliability of the measurements.

The inclusion of an interface that displays or communicates the determined arterial direction significantly enhances the usability of the device. Health professionals can use this information to adjust the device's placement, ensuring optimal alignment before starting a measurement.

Optionally, the device is configured to perform measurements at a sampling rate of at least 8000 Hz, preferably at least 10000 Hz, and wherein the first distance (D3) is at least 1 millimeter.

This allows for the detection of minute time shifts in PPG signal arrival times indicative of PWV. The photodiodes may be configured to sample at a rate sufficient to capture the short time shifts indicative of high pulse wave velocity, enhancing the precision of blood pressure readings.

The specified first distance (D3) of at least 1 millimeter allows for accurate time shift measurements at these high sampling rates, ensuring that the device can effectively resolve the small temporal differences important for accurate PWV calculation. This high sampling rate and specific spatial configuration collectively contribute to the device's capability to precisely monitor fluctuations in blood pressure, offering significant improvements over lower-resolution devices.

The device can operate at a high sampling rate of at least 8000 Hz, and preferably at least at 10000 Hz. This exceptionally high frequency can be important for capturing the rapid, transient changes in photoplethysmography (PPG) signals, which reflect blood volume changes due to arterial blood flow. The rapid pulsations of the arteries, especially those influenced by systolic and diastolic pressures, generate PPG signal fluctuations that occur in milliseconds. A higher sampling rate ensures that these fleeting events are captured with high resolution, which can be important for detecting the subtle variances in the PPG signals that are indicative of blood pressure changes.

The specified minimum first distance (D3) of at least 1 millimeter between the photodiodes in different groups can be important for effectively measuring the time shifts in PPG signals. This distance ensures that there is sufficient spatial separation to detect the slight temporal differences in the arrival of pulse waves at different photodiodes. These time shifts, although minimal, are important indicators of the velocity at which the pulse wave travels through the arterial system.

The high sampling rate combined with the specified spatial arrangement (first distance D3) allows the device to accurately calculate the PWV. By effectively resolving the quick PPG signal variations and the associated time shifts, the device can compute the PWV with a high degree of accuracy. PWV is a recognized metric for assessing arterial stiffness and is directly related to blood pressure; thus, precise PWV measurements lead to more accurate blood pressure readings. This integrated approach addresses and mitigates the inaccuracies found in devices with lower sampling rates and less optimized sensor configurations, which may not capture the full dynamics of arterial blood flow or may introduce errors in PWV calculations due to inadequate temporal resolution.

Optionally, the device is arranged to ensure a minimum spatial separation between photodiode groups, the minimum spatial separation being selected based on the sampling rate and desired sensitivity to pulse wave velocity changes.

This separation can be important for reducing noise and enhancing signal clarity, which in turn improves the accuracy of the PWV measurements. The chosen separation ensures that the device can effectively distinguish between signal changes due to pulse wave travel.

Maintaining an adequate spatial separation between photodiode groups minimizes the risk of signal interference and noise contamination. Photodiodes positioned too closely may capture overlapping signal reflections, which can confuse the distinction between signals from different arterial paths or tissue depths. Such overlapping would impair the device's ability to accurately determine the timing of pulse wave arrival at each sensor, an important factor in PWV calculations.

Furthermore, the spatial separation relates directly to the device's ability to resolve the time differences in pulse wave detection with high precision. A greater distance between groups allows for a clearer delineation of the time it takes for a pulse wave to travel from one group to the other. This clear delineation can be important for accurately calculating the PWV, as it hinges on the precise measurement of the time delay between two or more points along the path of the arterial blood flow.

In terms of the sampling rate, the distance between photodiode groups influences the resolution at which time differences can be detected. A higher sampling rate allows for finer resolution in timing measurements, which means that smaller distances may still yield reliable data. However, if the sampling rate is limited, a greater distance between sensors may be necessary to capture significant differences in pulse arrival times, thus ensuring that the measurements fall within the resolution capabilities of the system.

By selecting the appropriate minimum spatial separation based on these factors, the device ensures that each signal captured is distinct and clear, free from the effects of noise and interference that can otherwise compromise data quality. This careful configuration of sensor placement thereby enhances the overall reliability and precision of the PWV measurements, leading directly to more accurate and consistent blood pressure monitoring outcomes.

Optionally, the device is configured to adjust an intensity of one or more of the light sources based on photodiode feedback, wherein the mechanism is configured to change the intensity based on a value indicative of detected signal quality.

This capability allows for dynamic adjustment of light intensity, ensuring optimal signal quality across different tissue types and conditions. By adapting the light intensity in response to real-time data, the device can maintain high-quality PPG signal detection, which can be important for accurate PWV measurement and blood pressure monitoring.

The feedback mechanism within the blood pressure monitoring device can significantly enhance its operational effectiveness. This provides the ability to consistently maintain optimal signal quality despite variations in individual subject's tissue characteristics or external conditions that may affect signal clarity. Such conditions can include skin tone, tissue density, or even ambient light conditions, all of which may impact the penetration and reflection of light used to measure blood volume changes.

Variability in tissue characteristics, such as skin thickness, color, and the presence of hair, as well as external environmental factors like ambient light, can significantly impact the detection of PPG signals. By adjusting light intensity based on feedback from the photodiodes, the device can compensate for these variables, ensuring that the PPG signals remain robust and clear. This is achieved through an intelligent feedback loop within the device. The photodiodes act as sensors that continuously monitor the quality of the reflected light they receive, which includes PPG signals indicative of blood volume changes. If the signal quality falls below a certain threshold, possibly due to poor light penetration or excessive ambient light interference, the device automatically adjusts the intensity of the light sources to optimize signal clarity. This adjustment can mean increasing the intensity to overcome absorption or scattering issues in denser tissues, or reducing it to prevent saturation in lighter tissues or under strong ambient light conditions.

The dynamic adjustment enables to maintain a consistently high level of signal quality across a wide range of conditions and subjects. This reliability can be important for accurate PWV calculations, as precise timing of blood volume changes detected at different photodiode locations can be important. Improved signal quality directly translates to more accurate detection of these time differences, enhancing the overall reliability and precision of blood pressure measurements made by the device.

The implementation of this dynamic adjustment mechanism typically involves algorithms that can process input from the photodiodes in real-time, determining whether the current light intensity is adequate or needs adjustment. These algorithms are integrated into the device's processing unit, which controls the light sources accordingly. This feature not only maximizes the device's adaptability to varying physiological and environmental conditions but also minimizes the need for manual recalibration or adjustment by the user, thereby enhancing the user experience and device efficiency.

Optionally, the processor unit is configured to isolate photoplethysmography (PPG) waveforms attributable to deeper lying arteries from those attributable to superficial blood vessels for direct PWV measurement by utilizing the independent component analysis.

Optionally, the photodiodes are configured for high sampling rates, for example at least 5000 Hz.

Optionally, a shielding mechanism is arranged to reduce interference from ambient light and direct reflection.

Optionally, the device is capable of wireless communication with external devices for data transfer, analysis, and remote monitoring purposes.

Optionally, the device includes a user interface for displaying real-time blood pressure readings, historical data, and/or alerts based on the monitored blood pressure.

Optionally, the device is designed to perform periodic self-calibration using known reference measurements, ensuring long-term accuracy and reliability of blood pressure readings.

Optionally, the device incorporates machine learning algorithms to correlate PPG signal changes with blood pressure variations, allowing for periodic recalibration using standard blood pressure reference measurements.

Optionally, the device includes a mechanism for integrating ECG signals with PPG signals to enhance the accuracy of pulse wave velocity (PWV) measurement by utilizing the time difference between the electrical signal of the heart and the optical detection of the pulse.

Optionally, the device is configured to measure pulse arrival time (PAT) by detecting the time interval between the ECG signal and the PPG signal detected at a peripheral location, such as a finger or earlobe, to determine PWV and subsequently blood pressure.

Optionally, the device employs an array of photodiodes arranged to allow for the determination of arterial direction by analyzing spatial variations in an AC/DC ratio of the PPG signals.

Optionally, the device employs a spatial array of photodiodes and light sources to facilitate the determination of arterial direction by analyzing spatial variations in the AC/DC ratio of the PPG signals. This can assist in aligning the device's sensors with the main arterial flow for accurate PWV measurement.

Optionally, the device includes a dynamic light intensity adjustment mechanism based on photodiode feedback to optimize PPG signal quality across varying tissue types and environmental conditions. Advantageously, this can ensure reliable signal detection for accurate PWV measurement and blood pressure monitoring.

Optionally, the device incorporates various types of photodiodes, such as silicon, indium gallium arsenide, or perovskite photodiodes, to enhance the detection of PPG signals across different wavelengths and tissue depths. This can improve flexibility and adaptability in different measurement scenarios.

Optionally, the device integrates a signal quality assessment module that quantifies the quality of PPG signals based on parameters such as skewness, kurtosis, and signal-to-noise ratio, to ensure that only high-quality signals are used for PWV and blood pressure determination.

Optionally, the device is configured to utilize multiple wavelengths of light, including visible and near-infrared, to differentiate and separate PPG signals from superficial and deeper arteries, optimizing the detection of blood volume changes at various tissue depths.

Optionally, the device includes a shielding mechanism to reduce interference from ambient light and direct reflection, ensuring accurate PPG signal detection under varying environmental conditions.

Optionally, the processing unit is configured to apply multivariate signal processing techniques, such as principal component analysis (PCA) or independent component analysis (ICA), to isolate and enhance the detection of PPG signals attributable to deeper lying arteries, improving the accuracy of PWV and blood pressure measurements.

Optionally, the device employs a flexible array design that can be adapted to various body parts and anatomical differences, enhancing the versatility and applicability of the device for continuous, non-invasive blood pressure monitoring across different patient populations.

Optionally, the device can be configured to operate in different modes, such as continuous monitoring or spot-check measurements, to cater to various clinical and personal health monitoring needs.

According to an aspect, the invention provides for a method for monitoring blood pressure in a subject by analyzing tissue of the subject, the method comprising: emitting light towards a tissue of the subject using a plurality of light sources; detecting, with a plurality of photodiodes, light reflected from the tissue of the subject, wherein the reflected light includes photoplethysmography, PPG, signals indicative of blood volume changes, and wherein the photodiodes and light sources are organized into at least two groups, each group containing at least one light source and at least two photodiodes positioned at varying distances from the corresponding light source within the respective group to capture PPG signals from different tissue depths; processing the PPG signals received by the photodiodes to identify changes in blood volume, wherein the processing includes performing a signal separation analysis to separate PPG signals originating from varying depths within the tissue of the subject; calculating pulse wave velocity, PWV, based on the separated PPG waveforms associated with deeper arteries by analyzing the time difference between signal detections at the photodiodes positioned at varying distances; determining the blood pressure of the subject based on the calculated PWV by applying a predetermined relationship between PWV and blood pressure, wherein this determination includes correlating the PWV with known blood pressure values to estimate the blood pressure of the subject.

The method involves using multiple light sources to emit light towards the tissue and detecting the reflected light with an array of photodiodes organized into groups. The processing of these signals includes performing a signal separation analysis to distinguish between PPG signals from varying depths within the tissue, for accurately identifying the blood volume changes associated with different arterial structures. Calculating PWV by analyzing the time differences between these signals, and correlating PWV with known blood pressure values, allows for a precise determination of blood pressure. This method enhances the accuracy of blood pressure monitoring by providing a robust framework for analyzing complex signal data, ensuring reliable health assessments for subjects.

The photodiodes, organized into groups, capture PPG signals which are reflective of the pulsatile blood volume changes occurring within the tissue. These signals inherently contain data from multiple tissue depths, including both superficial and deeper arterial structures. The method utilizes signal separation analysis, such as independent component analysis (ICA), to distinctly identify and separate these PPG signals. This separation can be important as it isolates the specific signals that correspond to deeper arterial blood flow, which are important for accurate pulse wave velocity (PWN) calculations.

Once the PPG signals are separated by depth, the method involves calculating the PWV by analyzing the time differences in the arrival of these signals at the photodiodes positioned at varying distances. The PWV is an important parameter as it reflects the stiffness of the arteries, which is directly related to blood pressure. Accurately measuring the time it takes for the blood pulse to travel between two points provides an effective measure of PWV.

After calculating the PWV, the subject's blood pressure can be determined by applying a known relationship between PWV and blood pressure.

According to an aspect, the inventio provides for a blood pressure monitoring device comprising: at least two light sources configured to emit both visible and near-infrared light towards a subject's tissue to enable penetration at various depths within the tissue; a plurality of photodiodes arranged in at least two groups, with each photodiode positioned at a distinct distance from its respective light source, designed to detect reflected light from the subject's tissue, wherein the reflected light includes photoplethysmography (PPG) signals indicative of blood volume changes at different tissue depths; a processor equipped configured to process the PPG signals received by the photodiodes, wherein the processor isolates PPG signals corresponding to arterial blood flow from deeper lying arteries, separating these from signals originating from more superficial blood vessels; a calculation module within the processor, or as a separate component, designed to calculate pulse wave velocity (PWV) based on the time shift between the isolated PPG signals from deeper lying arteries as detected by the photodiodes across different groups, wherein the calculation accounts for the known distances between the photodiodes and their respective light sources, and utilizes the specific arrangement of photodiodes to accurately capture the travel path of the pulse wave; a blood pressure determination module, integrated within the processor or as an independent component, which determines the subject's blood pressure based on the calculated PWV, wherein this module utilizes a predetermined relationship between PWV and blood pressure to infer blood pressure non-invasively and continuously. Advantageously, the determination can be made through the analysis of the isolated PPG signals from deeper arteries and the accurately measured PWV.

It will be appreciated that any of the aspects, features and options described in view of the device apply equally to the method and the described system. It will also be clear that any one or more of the above aspects, features and options can be combined.

### BRIEF DESCRIPTION OF THE DRAWING

The invention will further be elucidated on the basis of exemplary embodiments which are represented in a drawing. The exemplary embodiments are given by way of non-limitative illustration. It is noted that the figures are only schematic representations of embodiments of the invention that are given by way of non-limiting example.

In the drawing:
Fig. 1 shows a schematic diagram of an embodiment of a device;
Fig. 2 shows a schematic diagram of an embodiment of a device;
Fig. 3 shows a schematic diagram of an exemplary pulse wave velocity measurement.
Fig. 4 shows a schematic diagram of an exemplary light probing;
Figs. 5a-d show a schematic diagram of exemplary embodiments of a device;
Fig. 6 shows diagrams with experimental results;
Fig. 7 shows diagrams with experimental results;
Fig. 8 shows diagrams with experimental results;
Fig. 9 shows diagrams with experimental results;
Fig. 10 shows diagrams with experimental results; and
Fig. 11 shows a schematic diagram of an exemplary method.

### DETAILED DESCRIPTION

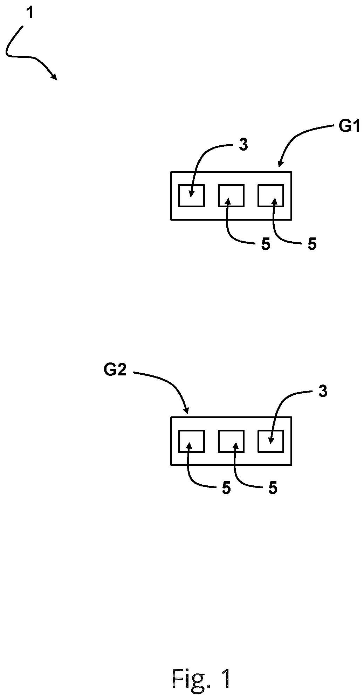

Fig. 1 shows a schematic diagram of an embodiment of a device 1 for monitoring blood pressure of a subject by performing measurements on a tissue of the subject. The device 1 comprises a plurality of light sources 3 and photodiodes 5, wherein the light sources 3 are configured to emit light and the photodiodes 5 are configured to detect light reflected from the tissue of the subject, wherein reflected light includes photoplethysmography, PPG, signals indicative of blood volume changes. The photodiodes 5 and light sources 3 are arranged into at least two groups G1, G2, each group G1, G2 comprising at least one light source 3 and at least two photodiodes 5 positioned at varying distances from a corresponding light source 3 within the respective group. The device further includes a processing unit configured to process the PPG signals received by the photodiodes 5, and wherein the processing unit is configured to perform signal separation analysis in order to separate PPG signals originating from different depths within the tissue of the subject. The processing unit is configured to calculate a pulse wave velocity (PWV) based on the separated PPG waveforms associated to deeper arteries by analyzing the time difference between signal detections at the photodiodes 5 positioned at varying distances. Furthermore, the processing unit is configured to determine a blood pressure of a subject based on the calculated PWV utilizing a predetermined relationship between PWV and blood pressure.

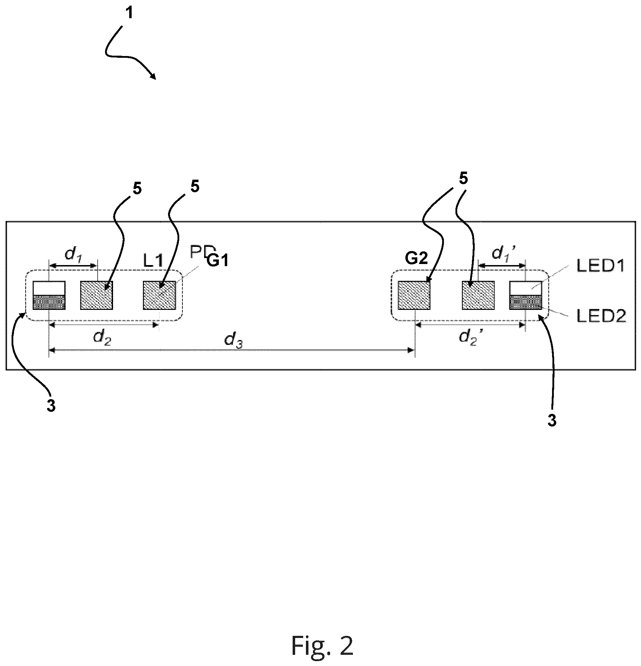

Fig. 2 shows a schematic diagram of an embodiment of a device 1. The device features multiple light sources 3 and photodiodes 5 that emit light and detect reflected signals. These components are organized into groups (G1, G2), each with one light source and multiple photodiodes at varied distances. A processing unit analyzes PPG signals from different tissue depths, computes pulse wave velocity (PWV) from the time delays in signal detection, and determines blood pressure based on PWV.

The arrangement of two groups of components: Group 1 (G1) and Group 2 (G2). Each group contains one light source 3 and two photodiodes 5. The distances between the components within each group and between the groups are labeled as follows:
- d1 and d'1: Distance between the light source 3 and the first photodiode in Groups G1 and G2, respectively.
- d_2 and d'2: Distance between the light source 3 and the second photodiode in Groups G1 and G2, respectively.
- d_3: Distance between the light source in Group G1 and the nearest photodiode in Group G2.

The layout is designed to allow for the measurement of pulse wave velocity by analyzing the time shift between the signals captured by the photodiodes in the different groups. This method aims to determine blood pressure by evaluating the velocity of blood pulse waves traveling between the deeper arteries, detected through photoplethysmography (PPG).

The system utilizes light sources that can emit both visible and near-infrared light, optimizing the detection capabilities for different tissue depths. In this example, each light source 3 has two LEDs, namely LED1 and LED2.

Various different configurations and arrangements are envisaged. The shown configuration has two light sources (3). However different number of light sources and different configurations may be employed. In this example, each group, G1 and G2, contains a light source that emits light in both the visible and near-infrared spectrum. The use of both light spectra allows for deeper penetration into tissue and the capture of PPG signals from both superficial and deeper arteries. It will be appreciated that his may be done in various alternative ways.

In this example, each light source is paired with two photodiodes positioned at different distances. These photodiodes are responsible for detecting light that is reflected back from the tissue, which contains information about blood volume changes within the tissue.

The distance d3 represents the space between the light source in Group G1 and the nearer photodiode in Group G2. This distance d3 is significantly larger than any distance d2 within each group G1, G2.

The configuration is set to measure the time difference in the arrival of the pulse wave at various photodiodes. Since the pulse wave velocity is a function of the arterial elasticity, which in turn is influenced by blood pressure, measuring the time shifts between these photodiodes can provide a direct estimation of the PWV.

In some examples, the device may be configured to operate at a high sampling rate, necessary to accurately detect the very short time delays between signal detections at different photodiodes, given the high speed of pulse waves (typically 5-10 m/s).

In some examples, the signals captured by the photodiodes are processed to separate the PPG waveforms originating from different tissue depths. This may be advantageously accomplished through Independent Component Analysis (ICA), a computational method used to segregate mixed signals into their constituent sources. - By analyzing the separated PPG waveforms, the device calculates the PWV. This calculation considers the known distances between the photodiodes and their respective light sources.

The precise arrangement allows the device to differentiate between the PPG signals contributed by deeper arteries versus those from superficial blood vessels. This is advantageous for monitoring the health of cardiovascular patients, as it provides a continuous, non-invasive, and accurate method to assess blood pressure.

The design's ability to isolate PPG signals from noise and other disturbances significantly enhances the reliability and accuracy of blood pressure measurements in an advantageous way.

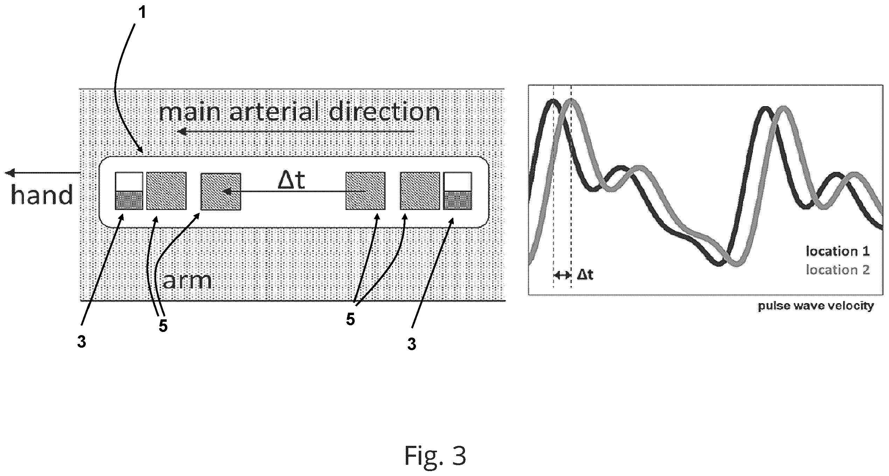

Fig. 3 illustrates pulse wave velocity measurement. Upon illumination with infrared light (with a higher penetration depth in tissue compared to visible light) independent component analysis reveals two distinct PPG waveforms originating from smaller blood vessels closer to the skin surface and from larger arteries deeper in the skin. The depth is not only achieved by using near-infrared, but also by increased PD-LED spacing . Unfortunately, the data from further distanced PDs may not result in good quality PPGs due to the strong attenuation of light through tissue. However, this can be resolved using ICA resulting in clear PPG waveforms. The visible light LED in combination with the nearest photodiode results in a high quality PPG signal close to the surface that can be used for reference and the same data can be used to determine the blood oxygenation (SpO2) when recorded at 2 wavelengths.

The accurate measurement and isolation of the PPG signal originating from deeper arteries is beneficial for PWV measurements:
1) Larger arteries in for example the outer limbs have a main travel direction along the arm and the pulse pathway more precisely determined.
2) The larger arteries branch into arterioles and those in turn branch into capillaries, all with less ordered directionality but also with less stretching of the arterial wall, resulting in a smaller AC component in the PPG signal.
3) The distinct PPG signals obtained by ICA show a larger difference than would be expected from a time shift due to pulse wave velocity. This implies determination of a pulse wave velocity from a single measured PPG signal at two locations is severely influenced by the actual local composition of this PPG signal from the PPG originating from deeper lying larger arteries and from more superficial smaller arteries.

It should be noted that the measurement array can have many different types of design configurations , but always limited by at least 2 groups (can be more) with individual light sources and a plurality of photodiodes and a spacing between the groups that allows for a time shift determination to obtain the PWV. The directionality between groups preferably follow the main arterial direction, as for example indicated in Fig. 3. Increasing the number of photodiodes allows for a better ICA, with a separation of common noise sources and/or separating even more than 2 distinct PPG signals when present. Below a number of these configuration are provided. A more special case is also presented in the larger array with a larger number of LEDs, that all together function as a linear light source along the array and where (in this particular configuration) many PDs in a single group are used for PPG measurements. In this case the directionality between the groups may be less clear and may heavily deviate from the main arterial direction, as long as the photodiodes used for PWV determination and their direction in between is known.

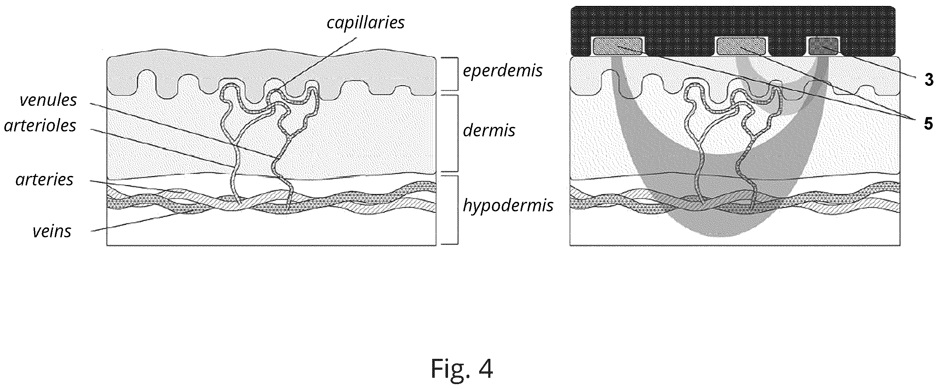

Fig. 4 shows a skin composition schematic with different blood vessels and an example how an increased LED-PD distance allows for deeper light probing in the tissue. The black layer on top is to indicate light shielding from direct reflected light off the skin surface and/or from ambient light and its fluctuations.

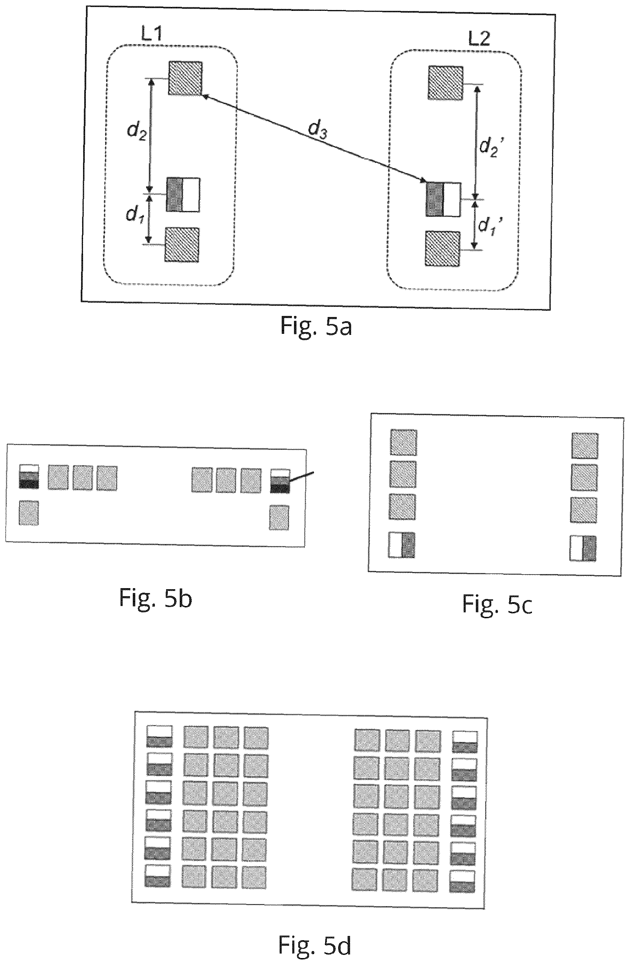

Figs. 5A-D show a schematic diagram of exemplary embodiments of a device 1. Different examples with different configurations are shown. In fig. 5A the photodiodes 5 are positioned on opposite sides of the light source unit in each group. In fig. 5B, each group has four photodiodes 5, and one light source unit. In this example, three photodiodes are positioned next to each other on one side of the light source unit, and one photodiode is positioned on another side of the light source unit, for each group. Other configurations and arrangements are also possible.

In the example shown in fig. 5B, the light source unit has three LEDs that can emit light with different properties.

In the exemplary configuration of Fig. 5C, two groups, each comprising a light source and three photodiodes is shown. Each light source emits both visible and near-infrared light and has two LEDs for this purpose. In each group, the photodiodes are positioned at different distances from their respective light sources. A first distance is defined as the distance between a light source in the first group of photodiodes and the nearest photodiode in a second group, wherein a second distance is defined as the distance between the furthest photodiode and the light source within that specific group, wherein the first distance is greater than any instance of D2 within either of the groups. The arrangement is designed to capture PPG signals at varying tissue depths, enabling the analysis of pulse wave velocity across different arterial segments by measuring the time differences between signal detections at various photodiode locations.

Fig. 5D shows a more extensive setup with numerous photodiodes arranged in a grid pattern, which could be used for more comprehensive tissue analysis, improving the accuracy of PWV and blood pressure estimations by capturing a broader range of signal interactions.

The large array of photodiodes (PDs) covers a broader area of the skin or tissue. This extensive coverage allows for the simultaneous capture of photoplethysmography (PPG) signals from multiple points over a significant portion of the body part, such as an arm or leg. With more PDs distributed across the array, the device can potentially receive data from various depths. Different PDs could be set to capture light at different wavelengths, thereby enhancing the penetration into deeper tissue layers and improving the detection of blood flow changes at these levels. By using a grid layout, the device can more accurately measure the time differences between the arrivals of the pulse wave at different PDs. This precision in capturing time shifts can be important for calculating the pulse wave velocity, an important determinant in non-invasive blood pressure monitoring.

The grid allows for the analysis of the pulse wave's directionality across the tissue. Understanding the pulse wave's path can help isolate the signals from major arteries, which are more relevant for accurate PWV measurements.

A larger number of PDs provides redundancy in signal capture, which is beneficial for filtering out noise and motion artifacts, common issues in wearable health monitoring devices.

With more data points, ICA can be more effectively employed to separate the mixed signals into distinct components. This separation can be important for distinguishing between the PPG signals from superficial blood vessels and those from deeper arteries.

The scalability of such an array means that the device can be adapted for different body sizes and specific medical needs. It can be customized for localized monitoring on small areas or expanded for more extensive coverage.

The array's versatility allows it to be used in various clinical and ambulatory settings, providing continuous monitoring with potentially higher reliability and less discomfort than traditional methods.

Hence, in some cases, the large array configuration provides a robust approach to enhancing the accuracy and reliability of blood pressure monitoring through spatially comprehensive data collection. This approach not only improves the measurement of PWV and subsequent blood pressure estimation but also enriches the data quality, which is important for developing more effective health monitoring systems.

It will be appreciated that the shown exemplary diagrams collectively emphasize the invention's capability to isolate and analyze PPG signals from varying depths within the tissue using a sophisticated arrangement of PDs and light sources, important for accurate and continuous blood pressure monitoring. The configuration allows for the extraction of distinct PPG waveforms necessary for determining PWV and, consequently, blood pressure, using signal processing techniques like independent component analysis (ICA).

### Exemplary Results

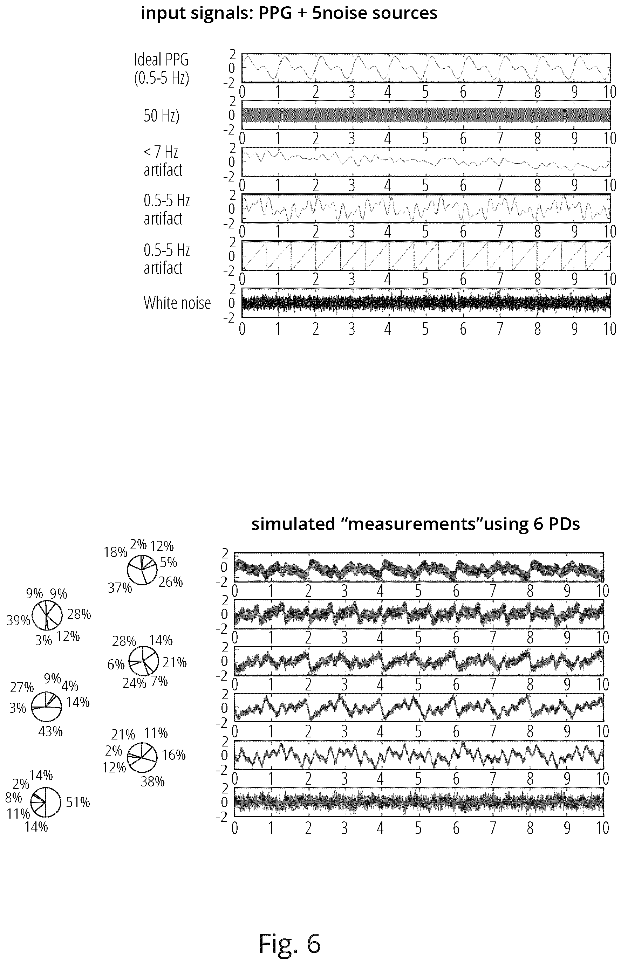

Fig. 6-10 show diagrams with experimental results. A mathematical PPG in combination with different sources of noise is employed. These were mixed in different ratio's resulting in 6 signals that represent measurements in parallel of 6 PDs. A total of 6 input signals, a mathematical PPG with 5 noise sources, mixed at different ratio's to simulate 6 parallel measurements is shown.

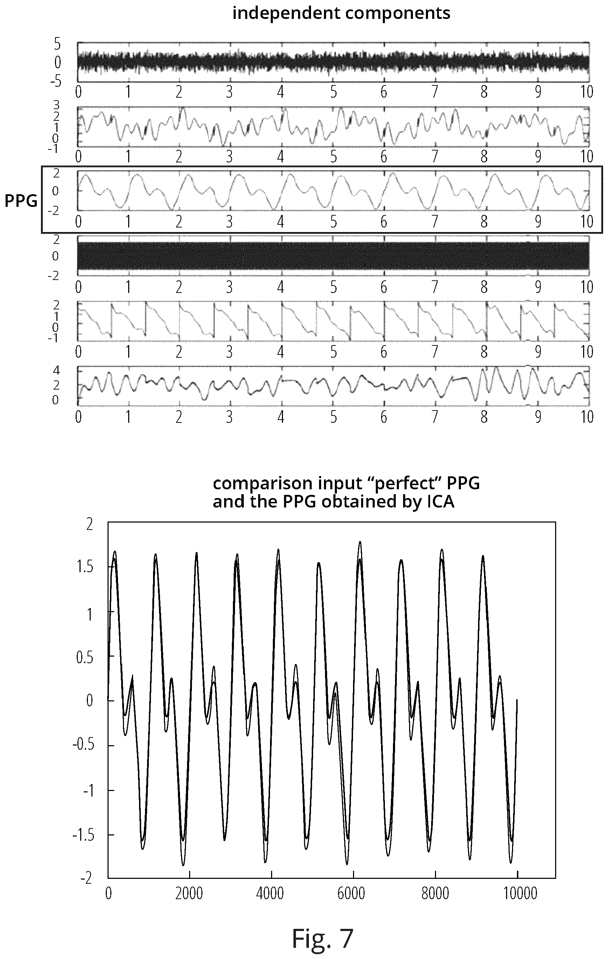

When ICA is applied to these 6 "measured" signals, 6 components are obtained that constitute these signals. These are shown in Fig. 7. In total 6 independent components are obtained by ICA of which one represents the PPG signal (boxed). It should be noted that the PPG signal obtained by ICA is, as expected, not identical to the mathematical PPG given as input. However, it clearly shows the excellent match which is much better than what could be obtained by filtering the different "measured" signals.

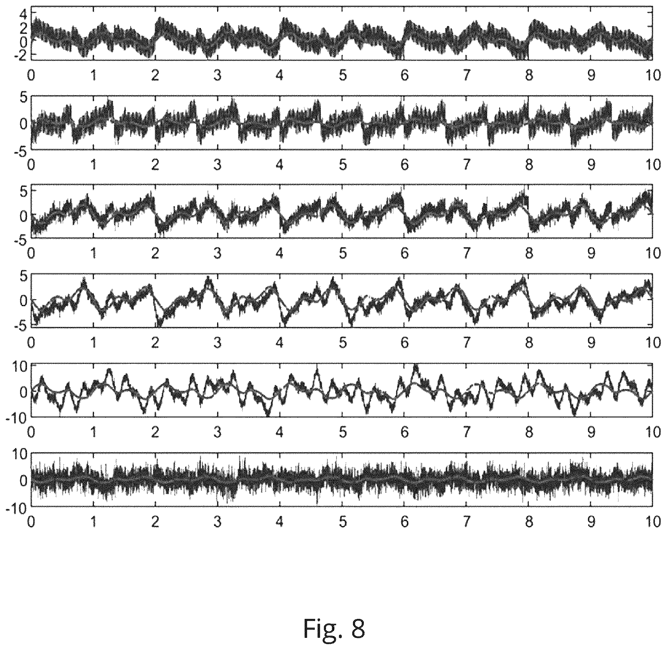

Fig. 8 shows PPG reconstruction using coefficients. The contribution of each of the components to each measurement can be determined, and here the component of the PPG signal is reconstructed for each measurement, showing that even when buried in the noise the ICA technique can reveal what the actual measured PPG signal was.

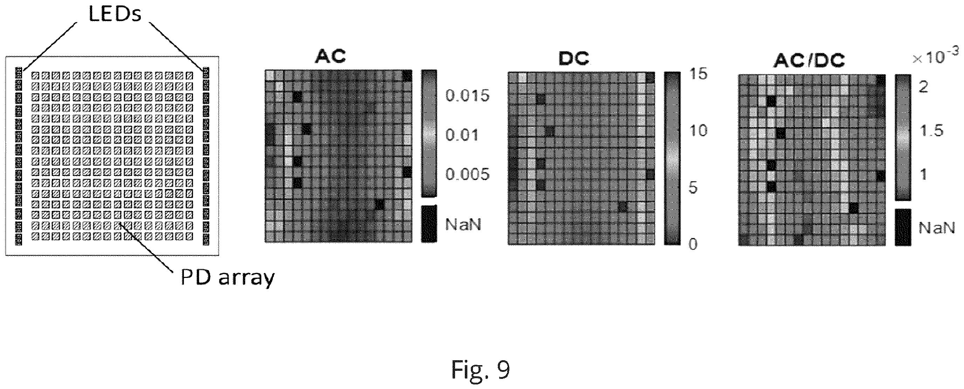

Fig. 9 shows mapping of AC, DC, and AC/DC ratio obtained on a photodiode array with side illumination. To show an increased depth probing with increasing LED-PD distance, a photodiode array was used with light sources at the edges. The AC and DC signal level both decrease towards the middle of the array (further away from the light sources), but the AC/DC ratio increases towards the middle, indicating an increased pulsatile component in the signal from deeper lying arteries.

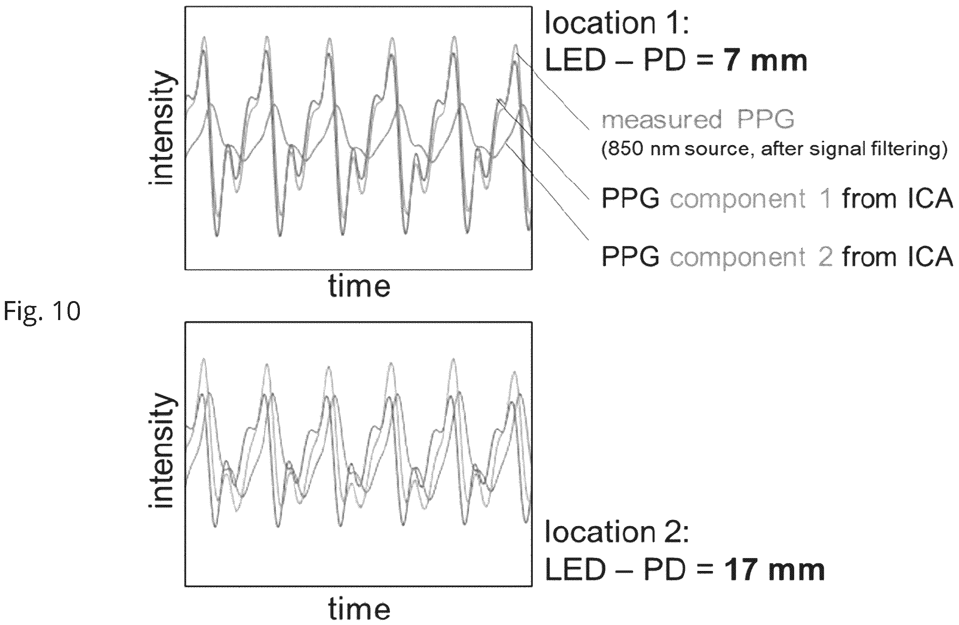

Fig. 10 shows measured PPG signals plotted together with the 2 PPG components resulting from ICA, for two different LED-PD distances. The independent component analysis from a reflective PPG measurement using a photodiode array with 850 nm illumination at the location of the brachial artery (upper arm) is shown in Figure 10. For two of the photodiodes the measured PPG signal is shown, with two distinct independent components originating from 2 distinct PPG signals. The PPG from deeper lying arteries has a much larger contribution the measured PPG signal (after filtering) when the PD-LED distance increases. It should be noted that the PPG from more superficial arteries will always be present in the signal since the light traveling to the photodiode from larger depths has to pass the more shallow depth regions of the skin twice. Logically using visible light and a close LED-PD distance only results in a single PPG component from ICA.

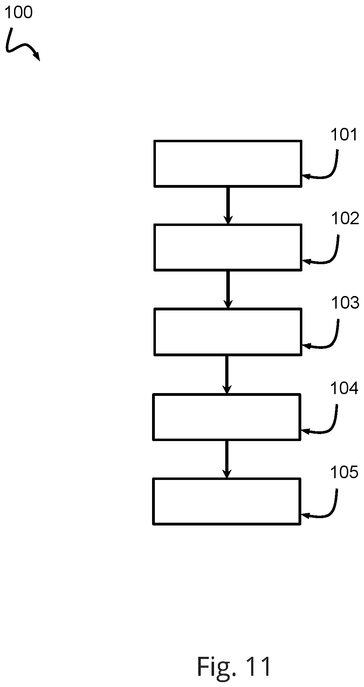

Fig. 11 shows a schematic diagram of an exemplary method 100 for monitoring blood pressure in a subject by analyzing tissue of the subject. In a first step 101, light is emitted towards a tissue of the subject using a plurality of light sources. In a second step 102, by means of a plurality of photodiodes, light reflected from the tissue of the subject, is detected, wherein the reflected light includes photoplethysmography, PPG, signals indicative of blood volume changes, and wherein the photodiodes and light sources are organized into at least two groups, each group containing at least one light source and at least two photodiodes positioned at varying distances from the corresponding light source within the respective group to capture PPG signals from different tissue depths. In a third step 103, the PPG signals are processed received by the photodiodes to identify changes in blood volume, wherein the processing includes performing a signal separation analysis to separate PPG signals originating from varying depths within the tissue of the subject. In a fourth step 104, pulse wave velocity, PWV, is calculated based on the separated PPG waveforms associated with deeper arteries by analyzing the time difference between signal detections at the photodiodes positioned at varying distances. In a fifth step 105, the blood pressure of the subject is determined based on the calculated PWV by applying a predetermined relationship between PWV and blood pressure, wherein this determination includes correlating the PWV with known blood pressure values to estimate the blood pressure of the subject.

Advantageously, the invention allows for a local PWV determination based on isolated PPG signals from deeper arteries of which main directionality is often known (or in case it's not at other body locations, which could always be determined in parallel by for example ultrasound, including artery diameter at that location). Furthermore, it eliminates the averaging of many unknowns into a single number as is commonly done for ECG-PPG pulse arrival time measurements.

In some examples, ICA is used with a specific device configuration, allowing for obtaining clean PPG signals, separate distinct PPG signals from different depth and even when the PPG signal has been largely attenuated by the tissue for large LED-PD distances and noise dominates the raw data, the PPG shape and its contribution to that signal can be determined.

In some examples, a configuration is employed with a minimum of 4 photodiodes with two light sources (each operating in the visible range and the near-infrared) to measure photoplethysmography (PPG) in reflection such that independent component analysis (ICA) can be used to isolate the PPG waveform of deeper lying arteries and the pulse wave velocity (PWV) through these arteries can be determined. Applying ICA data processing to a plurality of photodiodes for PPG analysis and 2 distinct PPG signals constituting together a measured PPG signal, provides for significant advantages.

In some examples, to accurately measure locally a pulse wave velocity by only using reflective PPG measurements with multiple photodiodes (PDs), the main challenge comes from the fact that the pulse wave velocity is very high (typically in the order of 5 -10 m/s) and time shifts between 2 PDs will be very short relative to the PPG signal period. For example, measuring a 10 m/s see pulse wave velocity over a distance of 10 cm for a person with a heart rate of 60 bpm will result in a PPG signal with a period of 1 see and a time shift of only 0.01 sec. This means that the sampling frequency should be high, the pulse pathway should be known, and the quality of the PPG signal should be very high, which is always influenced by motion artifacts and noise and filtering can only partly resolve these unwanted signal contributions.

It will be appreciated that the use of more photodiodes (more than 4) can provide more reliable results. The device configuration is such that 2 groups can be identified (indicated by G1 and G2 in for example fig. 2) where in each group two photodiodes have a different distance (d1 < d2) to the light source within that group. The groups are spaced such that the distance from the light source in group G1 to the nearest photodiode in group G2 is significantly larger than the distance between the furthest photodiode to the light source within a group (d3 >> d2).

It will be appreciated that the method may include computer implemented steps. All above mentioned steps can be computer implemented steps. Embodiments may comprise computer apparatus, wherein processes performed in computer apparatus. The invention also extends to computer programs, particularly computer programs on or in a carrier, adapted for putting the invention into practice. The program may be in the form of source or object code or in any other form suitable for use in the implementation of the processes according to the invention. The carrier may be any entity or device capable of carrying the program. For example, the carrier may comprise a storage medium, such as a ROM, for example a semiconductor ROM or hard disk. Further, the carrier may be a transmissible carrier such as an electrical or optical signal which may be conveyed via electrical or optical cable or by radio or other means, e.g. via the internet or cloud.

Some embodiments may be implemented, for example, using a machine or tangible computer-readable medium or article which may store an instruction or a set of instructions that, if executed by a machine, may cause the machine to perform a method and/or operations in accordance with the embodiments.

Various embodiments may be implemented using hardware elements, software elements, or a combination of both. Examples of hardware elements may include processors, microprocessors, circuits, application specific integrated circuits (ASIC), programmable logic devices (PLD), digital signal processors (DSP), field programmable gate array (FPGA), logic gates, registers, semiconductor device, microchips, chip sets, et cetera. Examples of software may include software components, programs, applications, computer programs, application programs, system programs, machine programs, operating system software, mobile apps, middleware, firmware, software modules, routines, subroutines, functions, computer implemented methods, procedures, software interfaces, application program interfaces (API), methods, instruction sets, computing code, computer code, et cetera.

Herein, the invention is described with reference to specific examples of embodiments of the invention. It will, however, be evident that various modifications, variations, alternatives and changes may be made therein, without departing from the essence of the invention. For the purpose of clarity and a concise description features are described herein as part of the same or separate embodiments, however, alternative embodiments having combinations of all or some of the features described in these separate embodiments are also envisaged and understood to fall within the framework of the invention as outlined by the claims. The specifications, figures and examples are, accordingly, to be regarded in an illustrative sense rather than in a restrictive sense. The invention is intended to embrace all alternatives, modifications and variations which fall within the scope of the appended claims. Further, many of the elements that are described are functional entities that may be implemented as discrete or distributed components or in conjunction with other components, in any suitable combination and location.

In the claims, any reference signs placed between parentheses shall not be construed as limiting the claim. The word 'comprising' does not exclude the presence of other features or steps than those listed in a claim. Furthermore, the words 'a' and 'an' shall not be construed as limited to 'only one', but instead are used to mean `at least one', and do not exclude a plurality. The term "and/or" includes any and all combinations of one or more of the associated listed items. The mere fact that certain measures are recited in mutually different claims does not indicate that a combination of these measures cannot be used to an advantage.

## Claims

1. A device for monitoring blood pressure of a subject by performing measurements on a tissue of the subject, comprising:
a plurality of light sources and photodiodes, wherein the light sources are configured to emit light and the photodiodes are configured to detect light reflected from the tissue of the subject, wherein reflected light includes photoplethysmography, PPG, signals indicative of blood volume changes, wherein the photodiodes and light sources are arranged into at least two groups, each group comprising at least one light source and at least two photodiodes positioned at varying distances from a corresponding light source within the respective group;
a processing unit configured to process the PPG signals received by the photodiodes, and wherein the processing unit is configured to perform signal separation analysis in order to separate PPG signals originating from different depths within the tissue of the subject;
wherein the processing unit is configured to calculate a pulse wave velocity (PWV) based on the separated PPG waveforms associated to deeper arteries by analyzing the time difference between signal detections at the photodiodes positioned at varying distances; and
wherein the processing unit is configured to determine a blood pressure of a subject based on the calculated PWV utilizing a predetermined relationship between PWV and blood pressure.

2. The device according to claim 1, wherein the signal separation analysis is a multivariate signal processing.

3. The device according to claim 1 or 2, wherein the signal separation analysis is an independent component analysis.

4. The device according to claim 1, 2 or 3, wherein the processing unit is configured to calculate the PWV based on time shifts between the separated PPG signals corresponding to arterial blood flow from deeper lying arteries as detected by photodiodes across different groups, and wherein the processing unit is configured to perform the calculation taking into account known distances between the photodiodes and their respective light sources in the different groups and/or specific arrangement of photodiodes in order to capture the travel path of the pulse wave.

5. The device according to any one of the preceding claims, wherein the device employs a plurality of light wavelengths in order to differentiate and separate PPG signals from superficial and deeper arteries based on the absorption characteristics of the tissue.

6. The device according to claim 5, wherein each light source is configured to emit light in both the visible and near-infrared spectrum.

7. The device according to any one of the preceding claims, wherein a first distance (D3) is defined as the distance between a light source in the first group of photodiodes and the nearest photodiode in a second group, wherein a second distance (D2) is defined as the distance between the furthest photodiode and the light source within that specific group, wherein the first distance is greater than any instance of D2 within either of the groups.

8. The device according to claim 7, wherein the first distance is at least twice the second distance.

9. The device according to any one of the preceding claims, wherein the device is configured to determine a value indicative of an arterial direction at or approximate a location where the device is configured to perform blood pressure monitoring, wherein the device includes an array of photodiodes arranged to allow for the determination of the arterial direction by analyzing spatial variations in an AC/DC ratio of the PPG signals.

10. The device according to any one of the preceding claims, wherein the determination of arterial direction is utilized to refine the calculation of PWV by ensuring a measurement path substantially aligns with a main directionality of arterial flow.

11. The device according to claims 9 or 10, wherein the device is configured to analyze PPG signals and spatial data from the photodiodes and light sources, to calculate the arterial direction at or approximate to the location on a body of the subject where blood pressure monitoring is performed, wherein the processing unit is configured to distinguishing PPG signal characteristics that vary with arterial blood flow direction, and employ a geometric analysis based on the relative positions of photodiodes and light sources to deduce the orientation of the underlying arteries, and wherein the device includes or is connectable to an interface for displaying or communicating the determined arterial direction in order to assist in placement and orientation of the device for blood pressure monitoring.

12. The device according to any one of the preceding claims, configured to perform measurements at a sampling rate of at least 8000 Hz, preferably at least 10000 Hz, and wherein the first distance (D3) is at least 1 millimeter.

13. The device according to any one of the preceding claims, wherein the device is arranged to ensure a minimum spatial separation between photodiode groups, the minimum spatial separation being selected based on the sampling rate and desired sensitivity to pulse wave velocity changes.

14. The device of any one of the preceding claims, wherein the device is configured to adjust an intensity of one or more of the light sources based on photodiode feedback, wherein the mechanism is configured to change the intensity based on a value indicative of detected signal quality.

15. A method for monitoring blood pressure in a subject by analyzing tissue of the subject, the method comprising:
emitting light towards a tissue of the subject using a plurality of light sources;
detecting, with a plurality of photodiodes, light reflected from the tissue of the subject, wherein the reflected light includes photoplethysmography, PPG, signals indicative of blood volume changes, and wherein the photodiodes and light sources are organized into at least two groups, each group containing at least one light source and at least two photodiodes positioned at varying distances from the corresponding light source within the respective group to capture PPG signals from different tissue depths;
processing the PPG signals received by the photodiodes to identify changes in blood volume, wherein the processing includes performing a signal separation analysis to separate PPG signals originating from varying depths within the tissue of the subject;
calculating pulse wave velocity, PWV, based on the separated PPG waveforms associated with deeper arteries by analyzing the time difference between signal detections at the photodiodes positioned at varying distances;
determining the blood pressure of the subject based on the calculated PWV by applying a predetermined relationship between PWV and blood pressure, wherein this determination includes correlating the PWV with known blood pressure values to estimate the blood pressure of the subject.
